# EUROPEAN PATENT APPLICATION

(11) **EP 3 210 569 A1**
(43) Date of publication of application: **30.08.2017**
(21) Application number: 15850016.5
(22) Date of filing: 09.10.2015
(51) Int. Cl.: A61F 2/12

(54) **IMPLANTABLE PROSTHETIC RING FOR CORRECTING AND PREVENTING BREAST AREOLA ASYMMETRY**

(30) Priority: 13.10.2014 ES 201400793
(71) Applicant: Kanouzi Baschour, Jack, 35017 Tafira Baja (Las Palmas de Gran Canaria) (ES)
(72) Inventor: Kanouzi Baschour, Jack, 35017 Tafira Baja (Las Palmas de Gran Canaria) (ES)
(86) International application number: PCT/ES2015/000135
(87) International publication number: WO 2016/059266

(57) **Abstract**

The invention consists on an absorbable prosthetic circular ring, for the correction and prevention of mammary areolas asymmetry. Its surgical implantation in the plane inferior to the areola allows reduction of traction and postsurgical tension exerted on the skin, thanks to a structure of hollow openings that permit uniform suturing of surrounding contiguous tissue, and that also reduces the risk of fibrosis by enabling passage of blood through. The structure is composed by an absorbable principal body (1) that consists on a single piece of circular surface (4), with hollow openings (2) trespassing completely and with vertical orientation the principal body. Its absorbable character avoids the necessity of secondary surgery for its extraction. Currently, there is no antecedent of any type of prosthesis designed for this purposes, being this unique and innovative invention in the sector of plastic surgery.

## Description

### Sector of the technique

The invention is framed in the technical sector of plastic surgery, more concretely in the relative to aesthetic and reconstructive mammary surgery.

### State of the technique

Currently, within the scope of plastic surgery, the mammary areolar asymmetry has acquired special relevance. There is no existence of any type of antecedent of prosthesis specifically designed for correction and prevention of mammary areolar asymmetry.

One of the techniques mostly performed, within the basic process of correction of asymmetry of the mammary areola consists only in suturing, without the implantation of any prosthesis that allows adjustment of mammary areolas to more exact and symmetric measurements.

### Explanation of the invention

The present invention refers to a new absorbable ring-shape prosthesis for correction of mammary areolar asymmetry, the description of its structure, principal characteristics, and advantages that its structure raises after the implantation procedure.

The present invention aims to achieve symmetry of the mammary areolas. The surgical implantation of this prosthetic ring, also allows to decrease the traction and postoperative tension exerted over the mammary skin and the areolas.

Various sizes are presented, adapting to individual characteristics of each patient. The standard measures of this prosthetic ring are 0.025m in diameter, with a principal body (1) of 0.003m of height, and 0.003m of width, or similar measures.

The advantage of counting on a composition of absorbable materials, avoids the necessity of secondary surgeries for the removal of the prosthesis after a fist implantation surgery. Its absorption may be prolonged up to 18 months.

To facilitate the process of absorption of the prosthesis ring, and cicatrizing of the surrounding tissues around it respectively, this invention presents a structure with hollow openings (2) that permit free flow of blood through it. The presence of hollow openings (2) with vertical orientation, 0.0015m of diameter, and every 0.004m of circumference of the prosthetic ring, provide great security, due to its considerable reduction of risk of tissue fibrosis.

The hollow openings (2) are useful also to be used as points of union and approximation in between borders of tissue where the incision was made initially and to the adjacent tissue; allowing that the tissue surrounding the prosthesis cicatrizes around the structure, maintaining the round shape of the areola. The tension will therefore be redistributed uniformly in the tissue sutured to the round prosthetic ring.

The size of each hollow opening (2) permits the passage of suture (3) through it, in the process of approximation of adjacent planes, utilizing point suturing.

The absorbable prosthetic ring will be implanted in the inferior plane to the areola and nipple, in between the mammary gland and subcutaneous tissue, without interrupting blood irrigation of the nipple, allowing its conservation, and minimizing risks of nipple necrosis. The material used in its composition will be metabolized gradually by the natural processes of the body, being present for sufficient time before its absorption, until correct cicatrizing is complete and adaptation to the new shape of the areola.

The secondary follow-up surgery to remove the implant is unnecessary, making this technology a valuable alternative for a wide variety of indications that include: mammary areolar asymmetry, surgical breast reduction, surgery of the tuberous breasts, mammary pexy, surgery for breast augmentation with implants, reconstructive surgery of the mammary areola utilizing autologous skin graft or similar.

The advantages of the use of this prosthetic ring are cited as follows. The correction of asymmetry of mammary areolas, achieving bilateral symmetry. Reduces tension of pulled skin, conserving the natural shape of the breast. Reduces the risk of stretch marks. Provides more stability, retaining and preventing from displacement, rotation and mobility of the mammary prosthesis implants for breast augmentation.

### Description of figures

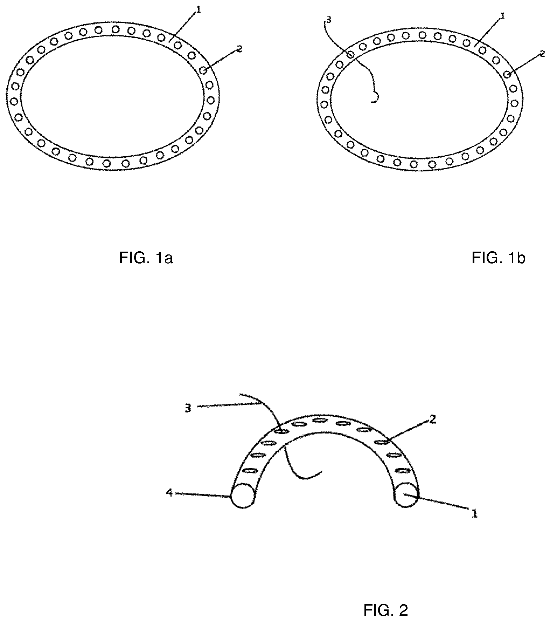

Figs. 1 a and 1 b show a superior view of the circular prosthesis with ring shape in accordance with the present invention. Fig.2 shows a lateral view with a plane that cuts the ring in half, that allows us to see the circular structure of the principal body of the prosthesis.
Fig.1 a shows the prosthesis of the present invention developed over a plane to see with more clarity the principal body (1) and the hollow openings (2) that cross through it.
Fig.2 shows the prosthesis of the present invention developed over a plane to see with more clarity the possibility of use of suture (3), that we can see introduced through one of the holes of the prosthesis.
Fig.2 shows the prosthesis of the present invention developed over a plane to see with more clarity the circular surface (4) of the principal body.

### Mode of realization of the invention

The mode of realization of the present invention is described next. The absorbable prosthesis is constructed with a circular ring-shape. Concretely, Figs.1 and 2 show a superior view where the principal body (1) and hollow openings (2) can be seen.

The principal body (1) of the prosthetic absorbable ring consists on a single piece of circular surface (4), with circular hollow openings (2), of 0.0015m of diameter, going through completely and with vertical orientation the 0.003m of height of the principal body, or similar measurements.

The hollow openings permit the passage of the suture (3) used to fix the adjacent tissue easily to various points of the prosthesis, managing to adopt the circular shape of the present invention after corresponding cicatrizing.

The prosthetic ring will be constructed with various sizes of diameter that are possible; individualizing the measures to the characteristics of the patient, and in relation to the size of the opposite areola. The standard diameter will be of 0.025m, or similar. The different measures will vary, starting from the 0.02m and increasing every 0.005m and up to 0.04m of diameter. That is, measures of the present invention built will be of 0.02m, 0.025m, 0.03m, 0.035m, and 0.04m of diameter respectively, or similar.

In terms of the consistency of the present invention, is highlighted the capacity of adopting a little elastic, reversible, deformation in which the principal body recovers its original shape when the force that produces the deformation is withdrawn; this permits an elevated resistance and prevents the absorbable prosthesis from breaking after an external traumatism or other elevated external pressure exerted over the prosthesis.

The absorbable prosthesis will be fabricated with shape of ring for the areola symmetry, with appropriated materials and its elements and components, using polylactic acid, polydioxanone, L-lactide-caprolactone, polyglycolic acid, or similar materials.

## Claims

1. Surgical prosthesis with circular ring shape for surgical treatment of the areolar mammary asymmetry, composed by a principal body (1), **characterized by** presenting:
multiple hollow openings (2), that trespass the principal body from one side to another, with vertical orientation, enabling the passage of flow through it, as well as of the sutures (3), for the correct cicatrizing and approximation of adjacent tissue to the prosthetic ring respectively.

2. Surgical prosthetic ring per claim 1, which consists of a structure of a circular ring in which the external pressure and tension exerted over the contiguous skin after its surgical implantation is transmitted to the principal body (1) of the prosthesis, redistributing therefore the tension of the contiguous skin sutured uniformly around the prosthetic ring. This permits maintenance of circular shape of the mammary areola and the contiguous sutured skin, adopting a shape similar to the prosthetic ring.

3. Prosthetic ring per claim 1, characterized because composition of the principal body is absorbable by the natural processes of the body, avoiding the necessity for secondary surgeries for extraction of the ring.

4. Prosthetic ring per claim 1, characterized for presenting a composition of the material with polylactic action, that stimulates the production of collagen, obtaining more regenerating effect in the process of subcutaneous cicatrizing after periareolar mammary surgery. This action stimulated regeneration of tissue and prevents of unwanted consequences of cutaneous superficial depressions of the areolar skin due to surgical incision produced in the surgical procedures related to the mammary areola, such as: correction of the asymmetry of mammary areola, breast reduction surgery, surgery of tuberous breasts, mammary pexy, surgery for breast augmentation with implants, reconstructive surgery of the areola and utilizing autologous skin grafts or similar.

5. Prosthetic surgical ring per claim 1, **characterized by** a structure able to adapt the mammary areola to millimeter measures larger or smaller than the preexisting, achieving the adaptation of the areolar size, with symmetric results of both mammary areolas, utilizing the preexisting present tissue or using autologous grafts.
